# EUROPEAN PATENT SPECIFICATION

(11) **EP 0 955 902 B1**
(45) Date of publication and mention of the grant of the patent: **13.10.2004**
(21) Application number: 98957446.2
(22) Date of filing: 30.10.1998
(51) Int. Cl.: A61B 17/00

(54) **HEMOSTATIC PUNCTURE CLOSURE DEVICE**
HÄMOSTATISCHE PUNKTIONSVERSCHLUSSVORRICHTUNG
DISPOSITIF HEMOSTATIQUE D'OBTURATION DE PERFORATIONS

(30) Priority: 31.10.1997 US 63881
(43) Date of publication of application: 17.11.1999
(73) Proprietor: St. Jude Medical Puerto Rico B.V., 1083 HK Amsterdam (NL)
(72) Inventor: ABRAHAMSON, Timothy, Alan, Seattle, WA 98178 (US)
(74) Representative: Strehl Schübel-Hopf & Partner
(86) International application number: PCT/US1998/023102
(87) International publication number: WO 1999/022646

(56) References cited:
- WO-A-93/07813
- WO-A-96/24290
- US-A- 5 021 059
- US-A- 5 222 974

## Description

### Field of the Invention

The present invention relates generally to a device to seal openings in the body of a patient and, more particularly, to an improved hemostatic puncture closure device which preferably includes an anchor, collagen member and a spring tensioned insertion and tamping member.

### Backcrround of the Invention

In United States Letters Patent No. 5,021,059 and U.S. Patent No. 5,222,974 granted to Kensey et al. there is disclosed a closure device and method of use for sealing a small incision or puncture in tissue separating one portion of the body of a living being from another portion thereof; e.g., a percutaneous puncture in an artery, to prevent the flow of a body fluid; e.g., blood, through the puncture. The closure device is arranged to be used with and deployed by an instrument which comprises a carrier or delivery instrument in the form of a tubular member. The tubular member has a proximal portion and a distal portion. The distal portion of the tubular member includes an open free end which is arranged to be introduced through the incision or puncture. The proximal portion of the tubular member is arranged to be located externally of the body of the human patient when the distal portion extends through the incision or puncture and into the blood vessel of the patient.

The closure devices of the Kensey patents generally consist of three basic sealing components; namely, an anchor member, a sealing member and a filament; e.g., suture. The sealing member is formed of a hemostatic material; e.g., compressed collagen foam and is configured to engage the tissue adjacent to the puncture. The anchor member is configured to pass through the puncture in one direction but is resistant to passage therethrough in the opposite direction. The filament is connected between the anchor member and the sealing member in a pulley-like arrangement so that they may be moved relative to each other by the application of a pulling force on the filament.

The delivery instrument is arranged to expel the anchor member through the puncture; e.g., into the artery, and to draw the tissue engaging portion of the anchor member into engagement with the tissue contiguous with the puncture. The filament extends through the instrument to a point outside the body of the patient and is arranged to be drawn in the proximal direction, whereupon the portion of the filament connecting the anchor member and the sealing member causes the tissue engaging portion of the sealing member to move with respect to the anchor member and into engagement with the tissue contiguous with the puncture on the opposite side thereof from the anchor member. This action causes the tissue engaging portion of the sealing member to seal the puncture from the flow of fluid therethrough.

The closure device and deploying instrument disclosed in Patent Nos. 5,021,059 and 5,222,974 may occasionally leave something to be desired from the standpoints of effectiveness and efficiency of use. For example, an initial measurement or locating step is performed to identify the location of the wall of the blood vessel and to ensure that the closure device and distal end of the delivery instrument are properly positioned with respect to the wall of the blood vessel prior to expulsion of the closure member.

Additionally, it is currently necessary to attach a leaf spring or similar type member to the suture between a metal band and the sealing member once the closure device is deployed to apply a constant tension to the closure device for a limited period of time.

### Summary of the Invention

Accordingly, it is a general object of this invention to provide a device which overcomes the disadvantages of the prior art.

It is a further object of this invention to provide a reliable system for quickly, easily, safely and effectively sealing a percutaneous puncture in a blood vessel within the body of a living being from another portion.

It is yet another object of the present invention to provide a self contained system which may be used for sealing punctures of various sizes.

These and other objects of this invention are achieved by the invention defined in claim 1. In an embodiment, the system generally includes a procedure sheath, a carrier device and a closure assembly. The puncture comprises a tract extending through tissue overlying a target organ such as a blood vessel. The closure assembly generally includes an anchor member, a sealing member and a filament member. The filament member is connected to the anchor member and passes through the sealing member. The procedure sheath consists of a tubular member having a distal free end arranged to be inserted into the puncture tract and through the puncture. The carrier member is arranged to be inserted through the introducer member to expel the anchor member therefrom and to draw the anchor member into engagement with the distal free end of the procedure sheath and wall of the blood vessel. The procedure sheath and the carrier member are movable together through the incision and into a portion of the blood vessel. The filament member extends from the proximal end of the carrier tube, into the puncture and through the sealing member to the anchor member.

In accordance with an embodiment of this invention the carrier device includes a bypass tube, a tamper tube and spring housing and a method of use to enable one to readily seal the puncture while minimizing the steps and manipulation required to position the closure member. In accordance with another embodiment of the present invention, the filament member, anchor member and sealing member are positioned in the carrier device and may be tensioned during placement and after placement without the use of extraneous components to effectively seal incisions or punctures of various sizes.

In accordance with another embodiment of this invention the system includes various indicators on the carrier device to signal to the user that the anchor member and sealing member have properly deployed in the puncture tract and blood vessel of the patient to reliably seal the puncture.

### Brief Description of the Drawings

Other objects and many of the attendant advantages of this invention will readily be appreciated as the same becomes better understood by reference to the following detailed description when considered in connection with the accompanying drawings wherein:

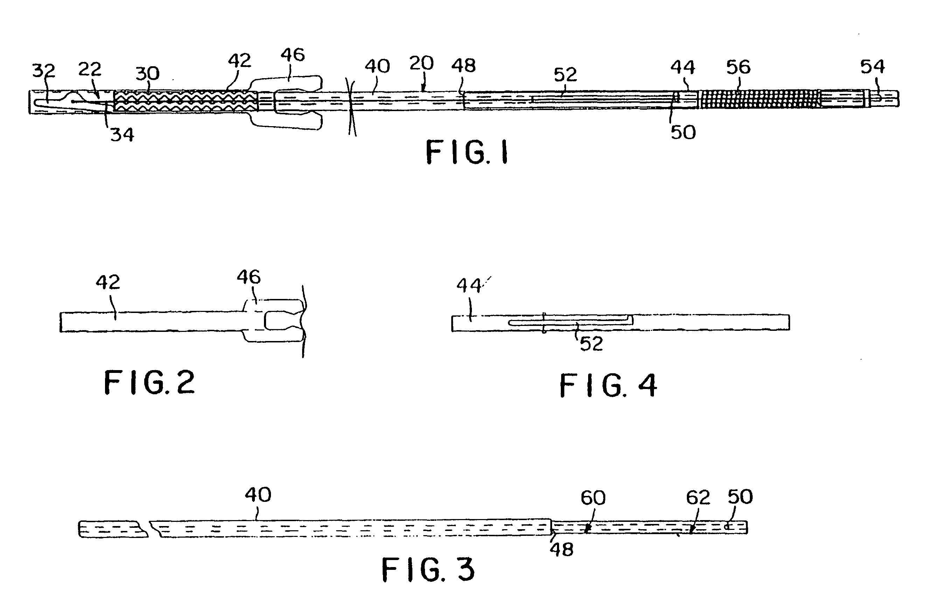

Figure 1 is a diagrammatic view, partially in cross section, of the assembled carrier device and closure device of the present invention;
Figure 2 is an enlarged side view, partially in cross section, showing the bypass tube of the present invention;
Figure 3 is an enlarged side view, partially in cross section, showing the tamper tube of the present invention;
Figure 4 is an enlarged side view, partially in cross section, showing the spring housing of the present invention;
Figure 5 is an enlarged side view, partially in cross section, showing the assembled closure device positioned in the bypass tube of the present invention;
Figure 6 is an enlarged side view, partially in cross section, showing the assembled proximal end portion of the tamper tube and the spring housing of the present invention;
Figure 7 is an enlarged side view, partially in cross section, showing the carrier device and closure device aligned with the introducer sheath of the present invention;
Figure 8 is an enlarged side view, partially in cross section, showing the bypass tube of carrier device of the present invention initially positioned within the introducer sheath;
Figure 9 is an enlarged side view, partially in cross section, showing the tamper tube of carrier device of the present invention initially moved further distally within the introducer sheath;
Figure 10 is an enlarged side view, partially in cross section, showing the tamper tube and spring housing of carrier device of the present invention twisted with respect to each other to release the pin from the groove and activate the tension of the spring member on the anchor member;
Figure 11 is an enlarged side view, partially in cross section, showing the advancement of the tamper tube of carrier device of the present invention to expel the anchor member from the introducer sheath and expose the stop marker on the tamper tube;
Figure 12 is an enlarged side view, partially in cross section, showing the proximal movement of the carrier device of the present invention in the puncture to cause the engagement of the anchor member along the wall of the blood vessel;
Figure 13 is an enlarged side view, partially in cross section, showing the tamper tube and spring housing of Figure 12 with the introducer sheath and bypass tube removed from the puncture;
Figure 14 is an enlarged side view, partially in cross section, showing the tamper tube and spring housing of the present invention with the second marker on the tamper tube exposed; and
Figure 15 is an enlarged side view, partially in cross section, showing the closure device of the present invention deployed in the puncture and blood vessel of the patient.

### Detailed Description of the Preferred Embodiment

Referring now in greater detail to the various figures of the drawings wherein like reference characters refer to like parts, there is shown at 20 a carrier device forming a portion of a system for deploying a closure device 22 to seal a percutaneous puncture 24 within a blood vessel 26; e.g., the femoral artery, constructed in accordance with this invention. The puncture 24 includes not only the opening in the wall of the vessel but also the tract; i.e., the passageway in the tissue located between the vessel and the skin of the human patient which is formed when the vessel is punctured. As used herein, the distal end of an element is referred to as the end of the element nearest to the patient and the proximal end of an element is referred to as the element furthest from the patient.

The carrier device 20 and closure device 22 have particular utility when used in connection with various intravascular procedures, such as angiography, cardiac catheterization, balloon angioplasty and other types of cardiovascular procedures, etc. since the closure device 22 is designed to cause immediate hemostasis of the blood vessel; e.g., arterial, puncture. However, it is to be understood that while the description of the preferred embodiment instrument and closure contained herein is directed to the closure of percutaneous incisions or punctures in arteries, they have much more wide-spread applications, wherein it is desirable to close an opening in the body of the patient. Thus, the sealing of a percutaneous opening in an artery shown herein is intended to be merely exemplary.

Before describing the closure device 22 and the carrier device 20 for inserting it to seal the opening, a brief description of a typical, conventional, intravascular surgical procedure; e.g., catheter instrumentation of an artery, utilizing a percutaneous opening will be given to best appreciate the features of the invention. In such a procedure a cannula of an instrument, such as an angiographic needle (not shown), is inserted percutaneously through the skin into the artery, such as the femoral artery, at the site of the instrument's insertion. The needle cannula is held in place, and the flexible end of a guide wire (not shown) is then passed through the cannula into the artery to the desired depth (i.e., longitudinal position therealong). Once the guide wire is in place, the needle cannula is removed, leaving the guide wire in place. An introducer sheath 28 and an arterial dilator (not shown) are then passed over the guide wire, through the puncture or incision and into the artery. The guide wire and then the dilator are removed leaving the introducer sheath in place. A catheter, or other intravascular instrument (not shown) is then inserted through the introducer sheath 28 and threaded down the artery 26 to the desired intravascular location; e.g., the site of the atherosclerotic occlusion.

Once the intravascular procedure (e.g., angiography, angioplasty or stent placement) has been completed, the catheter is removed. Thereafter, the sheath is removed and the surgeon or other trained person previously applied manual, digital pressure to the percutaneous puncture until hemostasis occurred. The standard of care for puncture hemostasis for many years was to apply digital or mechanical pressure on the puncture site for twenty minutes to an hour, depending on the puncture size and the degree of anticoagulant therapy. Obviously, this resulted in a significant amount of wasted time for the physicians and other catheter lab personnel and caused unnecessary inconvenience and discomfort for the patient. In addition, serious complications may arise from persistent bleeding and hematoma formation.

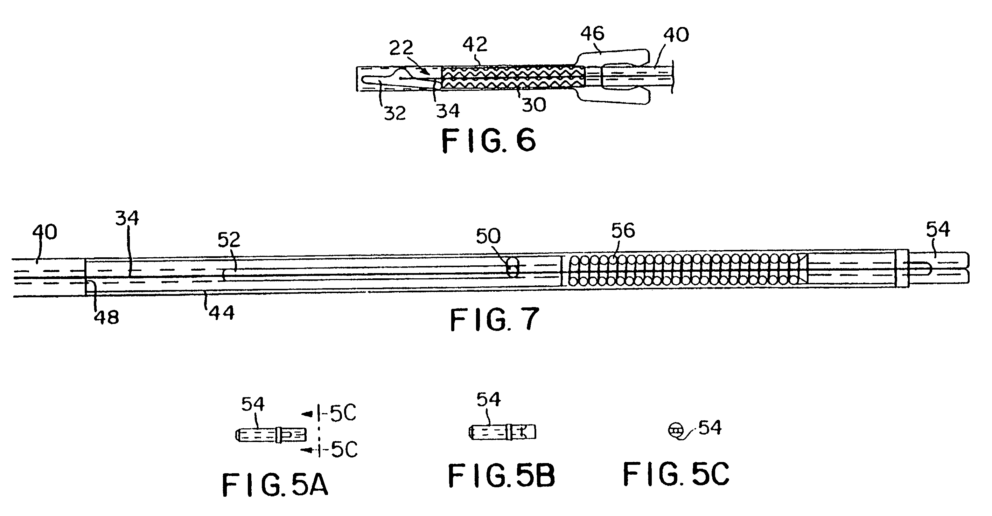

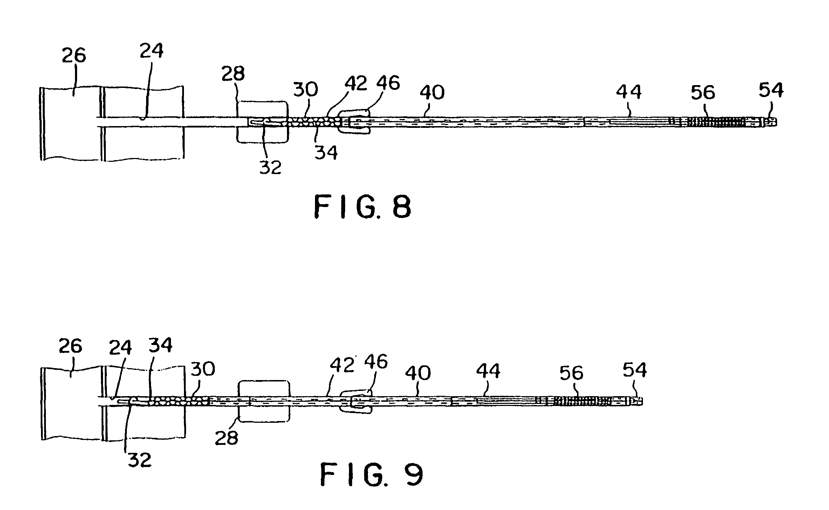

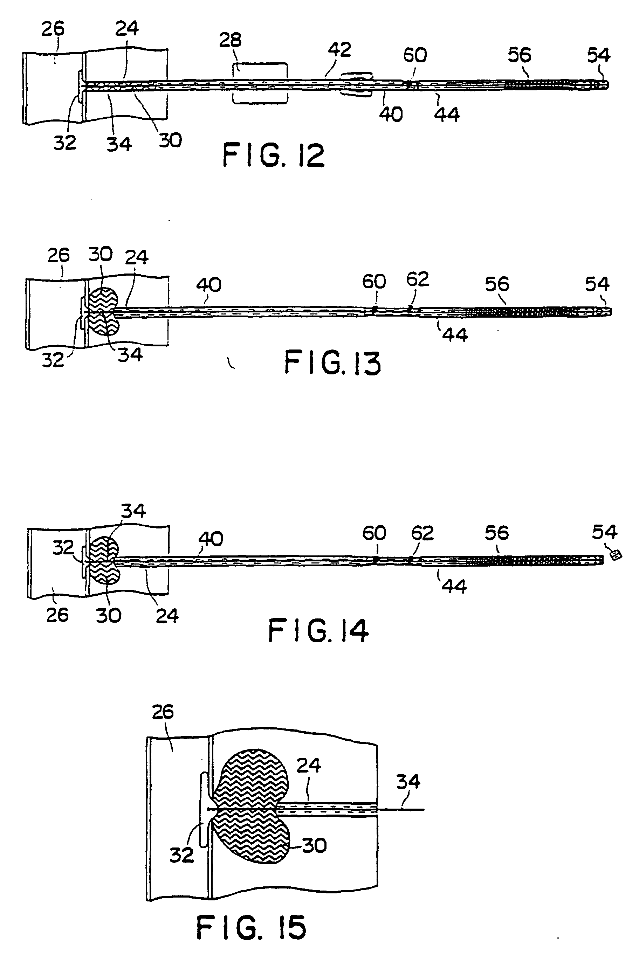

In accordance with the preferred method of use of this invention, the introducer sheath 28 remains in the artery and is moved so that its distal end is at a desired position therein, as will be described. Figures 7-14 are illustrations showing the sequential steps in the use of the carrier device of the present invention to deploy the closure device to seal the percutaneous puncture in the blood vessel of the human or animal patient. The carrier device 20 having the closure device 22 therein is initially inserted into the introducer sheath. The closure device is then deployed (ejected) and operated to immediately seal the arterial puncture site 24 and plug the tract. Moreover, as will be appreciated from the description to follow, the closure device 22 is designed to reduce post-procedure puncture complications, cause minimal inflammatory reaction and resorb completely within a relatively short period of time; e.g., sixty to ninety days.

The sealing member 30 is preferably formed as a cylindrical member which is made of a compressible, resorbable hemostatic or clot promoting material such as collagen. The sealing member 30 is initially compressed from a larger diameter configuration to a smaller diameter, elongated member which is inserted into the carrier device 20. The preferred diameter of the sealing member is relatively small, e.g. about 2.52 mm to be suitable for use within a carrier device which has an outer diameter of between about 4 french to 10 french depending on the size of the insertion sheath used in the procedure.

The anchor member 32 basically comprises a thin, narrow strip or bar of a resorbable and preferably moldable material such as a resorbable lactide/glycolide polymer manufactured and sold by various companies such as Medisorb Technologies International L.P. under the trade designation MEDISORB. The strip is sufficiently rigid such that once it is in the desired position in the artery (as will be described later) it is relatively resistant to deformation to preclude it from accidentally bending or folding and passing back through the puncture through which it was first introduced. The anchor member 32 preferably has a generally planar top and bottom surface and a peripheral side surface. Each end of the anchor member 32 is rounded. The top surface of the anchor member preferably includes the end of the filament member molded therein or tied thereon. Alternately, a longitudinally extending slot may be formed in the anchor member and disposed perpendicularly to the top surface of the anchor member 32. A portion of a positioning or filament member 34 may then be threaded therethrough. In the form of the sealing member wherein the filament member 34 is threaded therethrough, a portion of the filament member 34 is threaded through a slot in the anchor member 32 and back out of the slot on the other side thereof. The filament member is then preferably tied in a knot near the proximal end portion of the sealing member 30 to connect the sealing member 30 to the anchor member 32.

The filament member 34 of the closure device 22 serves to interconnect the sealing member 30 and the anchor member 32 and to assist in the movement of the sealing member 30 toward the anchor member 32, once the anchor member 32 is in its desired position in the artery adjacent to the puncture. In accordance with a preferred embodiment of this invention, the filament member 34 or filament is formed of resorbable, flexible material such as a resorbable suture.

As can be seen in the drawings, the carrier device 20 is preferably formed of a somewhat flexible material, such as polyethylene or TEFLON, so that the carrier device 20 may be freely passed through the introducer sheath 28 into an operative position within the patient's artery, notwithstanding any slight curvature or bending of the introducer sleeve which may exist through the puncture or artery. The outside diameter of the carrier device 20 is preferably between about 6 to 12 French to conform with the size of the punctures formed by the majority of procedures and introducer sheaths. The carrier device 20 generally consists of an elongate and cylindrical tamper tube 40 and a shorter, cylindrically shaped bypass tube 42 slidably mounted on the distal end of the tamper tube 40. A spring housing 44 is provided along the proximal end of the tamper tube 40. The use of the bypass tube 42 enables the carrier device 20 to be inserted through a conventional hemostasis valve (not shown) formed on the proximal end portion of the introducer sheath 28 without damaging the closure device 20 of the present invention. The length of the tamper tube 40 is preferably chosen so that the distance between the distal end portion of the tamper tube 40 and a shoulder surface 48 thereon is sufficient to expel the anchor member 32 of the closure device from the distal end of the introducer sheath 28.

The closure device 22 is preferably preloaded within the bypass tube 42 of the carrier device 20 and is positioned distally of the tamper tube 40. As shown, the anchor member 32 is preferably oriented longitudinally within the distal end portion of the bypass tube 42 and is positioned laterally of the central longitudinal axis of the carrier device 20. The sealing member 30 is located within the majority of the bypass tube 42 and just behind (proximally) the anchor member 32. The sealing member preferably fills the entire longitudinal cross section of the bypass tube 42. The filament member 34 extends from the anchor member 32, through the longitudinal axis of the sealing member 30 and tamper tube 40 and to the proximal end of the spring housing 44 where it is engaged by a suture lock 54. The bypass tube 42 may also include reference wings 46 or detents (not shown) along its periphery. The reference wings 46 serve as a visual guide to help the user orient the carrier device 20 to a proper yaw angle with respect to the introducer sheath 28 as will be described later.

The distal end of the tamper tube 40 slidably fits within the proximal end portion of the bypass tube 42 and is in contact with the sealing member 30 of the closure device 22. The proximal end of the tamper tube 40 extends into an opening in the distal end portion of the spring housing 44 and includes an outer circumferential shoulder surface 48 on the proximal end portion thereof. The tamper tube 40 is movably secured to the spring housing 44 by any suitable means, including the pin 50 and groove 52 arrangement shown in the preferred form of the present invention. In the preferred embodiment of the present invention, the length of the groove is preferably the same as or longer than the length of the anchor member 32 and sealing member 30. A tension force is generated by the interaction of the tamper tube 40 and the spring housing 44. A preferably coiled and compressed spring 56 is located within the spring housing 44. The suture lock 54 is fixedly attached to the proximal end of the spring housing 44 and contacts the proximal end of the coiled spring 56 to maintain the desired tension and compression in the coiled spring 56. The distal end of the coiled spring 56 is preferably in contact with the proximal end of the tamper tube. As will be appreciated by those skilled in the art, the tensioning assembly just described is intended to provide a predetermined amount of tension on the filament member 34 and may be actuated by various methods, including the relative rotation of a pair of members, a latch or button release mechanism or other available methods to actuate the tensioning assembly as desired by the user.

The preferred method of use for the present invention includes the initial step of positioning the distal end introducer sheath 28 in the artery a short distance beyond the wall of the blood vessel. This may be accomplished in a variety of ways, including through the use of an artery locator device as disclosed in U.S. Patent No. 5,222,974. After the introducer sheath is properly positioned in the artery, the introducer sheath 28 must be kept fixed with respect to the skin and blood vessel of the patient.

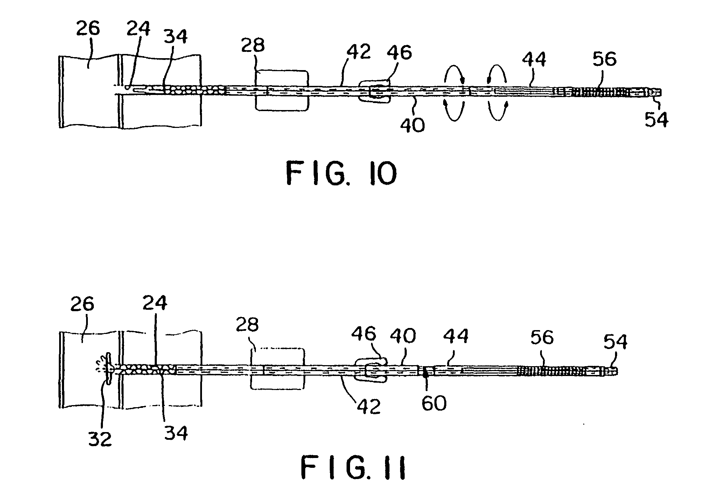

The physician initially grasps and fully inserts the bypass tube 42 portion of the carrier device 20 into the previously positioned introducer sheath 28. On complete insertion of the bypass tube 42 into the introducer sheath 28, the tamper tube 40 is advanced further into the introducer sheath 28 so that the anchor member 32 of the closure device 22 is positioned a short distance proximally of the distal end of the introducer sheath 28. As shown in Figure 10, the tamper tube 40 and spring housing 44 are grasped and twisted with respect to each other to release the tension lock created by the groove 52 and pin 50 to release the compression on the coiled spring 56 such that the pin 50 is moved from the proximal locked portion of the groove 52 to the distal portion of the groove 52. The release of the compression on the coiled spring 56 tensions the filament member 34 between the suture lock 54 and the anchor member 32 in the bypass tube 42. The tamper tube 40 is then grasped and moved distally with respect to the bypass tube 42 and introducer sheath 28 to move the anchor member 32 out of the distal end of the introducer sheath 28. Because the filament member 34 is tensioned by the coiled spring 56, as the anchor member 32 passes beyond the distal end of the introducer sheath 28 it is automatically toggled and deploys into the blood vessel generally adjacent to the wall of the artery and puncture. The application of the tension on the anchor member 32 also causes the spring housing 44 to move proximally with respect to the tamper tube 40 to expose a stop marker 60 on the body of the tamper tube 40 when the anchor member 32 is deployed. The stop marker 60 provides a positive indication to the user that the anchor member has been deployed. The user then withdraws the carrier device 20 with respect to the introducer sheath 28 and bypass tube 40 until resistance is encountered. The existence of resistance to continued withdrawal indicates that the anchor member 32 has engaged the wall of the blood vessel.

The introducer sheath 28 and bypass tube 42 are then removed from the puncture by passing the introducer sheath 28 and bypass tube 42 over the tamper tube 40 and spring housing 44. As the introducer sheath 28 and bypass tube 42 are removed, the coiled spring 56 continues to apply a tamping force to the closure device 22. The distal end portion of the tamping tube 40 pushes against the proximal end of the sealing member 30 so that as the sealing member 30 absorbs fluids from the surrounding tissue and softens, the sealing member 30 is transformed from the generally cylindrical configuration it formed when it was positioned in the tamper tube 30 to a more bulbous and malleable shape which conforms to the tissue surrounding the puncture. As the sealing member 30 softens and presses against the tissue adjacent to the blood vessel, the tamping member 40 gradually moves distally in the puncture and the coiled spring 56 in the spring housing 44 causes relative movement between the tamper tube 40 and the spring housing 44 so that a second marker 62 is exposed on the outer surface of the tamper tube 40. Exposure of this second marker 62 indicates to the user that the sealing member 30 of the closure device 22 has been compressed sufficiently by the continued tension on the filament member 34 to provide reliable closure of the puncture. If desired, a few gentle compactions with the tamper tube 40 may also be applied to assist the sealing member 30 to conform to the tissue adjacent to the puncture and to assist in the frictional engagement of the filament member 34 by the sealing member 30. It is believed that initial hemostasis occurs very quickly, thereby locking the closure device 20 in position in the puncture. It should be noted that during this additional tamping action care must be taken to maintain tension on the filament member 34 at a load equal to or greater than that used on the tamper member 40 to ensure that the tamping action doesn't propel the sealing member 30 into the interior of the blood vessel.

Once the second marker 62 is visible, the user may then cut, clip or otherwise cause the suture lock 54 to release the filament member 34. When the filament member has been released from the suture lock 54, the tamper tube 40 and spring housing 44 may be removed and then the filament member 34 may be cut at the skin level of the patient. The closure device 22 is now initially locked in place through the clotting of the hemostatic sealing member 30 and by tension between the sealing member 30 and the anchor member 32 as well as the interaction between the sealing member 30 and the tissue surrounding the puncture. Thus the artery wall is sandwiched between the sealing member 30 and the anchor member 32. Within a few hours after deployment, the anchor member 32 will typically be coated with fibrin and thus attached firmly to the arterial wall, thereby eliminating the possibility of distal embolization. After approximately thirty days, only a small deposit of anchor material will remain. In fact, resorption of all components will have typically occurred after approximately sixty days.

As should also be appreciated from the foregoing, the closure device, the carrier device for deploying it and their method of use enables the ready, effective and efficient sealing of a percutaneous puncture in an artery. Thus, it is expected that the present hemostatic puncture closure device will be a significant advancement in the fields of cardiology and radiology. The present device may allow continuance of anticoagulation post-procedure, more aggressive use of thrombolytic agents and safer use of large bore catheters. It should also reduce discomfort and complication rates for patients, allow many in-patient procedures to be performed safely on an out-patient basis, decrease the time and cost of interventional procedures and reduce exposure of hospital personnel to human blood.

Without further elaboration, the foregoing will so fully illustrate our invention that others may, by applying current or future knowledge, adopt the same for use under various conditions of service.

## Claims

1. An assembly for sealing an incision or puncture in the body of a patient wherein the puncture extends from the skin of the patient into a blood vessel (26), duct or lumen of the patient, the assembly comprising:
a first member (32) formed of a bioabsorbable material and sized to be positioned and anchored in the blood vessel (26), duct or lumen of the patient,
a second member (30) formed of a bioabsorbable material for sealing the puncture from the flow of fluids therethrough in combination with said first member (32),
a filament member (34) formed of a bioabsorbable material and including a portion extending proximally of said first member (32) and through said second member (30), thereby interconnecting said first member (23) and said second member (30), and
a carrier device (20) including a tensioning member (56) formed as a part thereof, said carrier device accommodating said first member (32), said second member (30) and said filament member (34) therein,
**characterised in that** said tensioning member (56) is operatively connected to said filament member (34) to apply tension thereto and cause said first member (32) to enter and come into contact with the wall of the blood vessel (26), duct or lumen to be sealed upon deployment of said first member (32) and said second member (30) from said carrier device (20).

2. The assembly of claim 1 further including a tamper member (40), wherein said tensioning member (56) applies compression forces to said tamper member (40) to cause said second member (30) to move distally in the puncture towards said first member (32).

3. The assembly of claim 1 wherein said filament member (34) is threaded through said second member (30) and is frictionally engaged with at least one of said first member (32), said second member (30) and said tensioning member (56).

4. The assembly of claim 1 wherein said carrier device (20) further includes a tamper member (40) and said filament member (34) is slidable through said tamper member (40).

5. The assembly of claim 1 further including an elongate tamper member (40) through which said filament member (34) is threaded.

6. The assembly of claim 1 further including a spring housing (44) retaining said tensioning member (56).

7. The assembly of claim 6 wherein said filament member (34) is threaded through at least a portion of said spring housing (44).

8. The assembly of claim 1 further including an elongate spring housing (44) and an elongate tamper member (40), wherein said tensioning member (56) is retained in said spring housing (44) and is oriented to cause distal movement of said tamper member (40) with respect to said spring housing (44).

9. The assembly of claim 8 wherein rotation of said spring housing (44) with respect to said tamper member (40) causes the application of a force moving said tamper member (40) distally in the puncture in contact with said second member (30).

10. The assembly of claim 1 wherein said carrier device (20) further includes an elongate spring housing (44) and an elongate tamper member (40), wherein said spring housing (44) and said tamper member (40) are oriented with respect to each other to have a first length with said tensioning member (56) being retained in said spring housing (44), and permitting the movement of said spring housing (44) and said tamper member (40) to a second length which is greater than said first length.

11. The assembly of claim 10 wherein movement of said spring housing (44) and said tamper member (40) from said first length to said second length is caused by a release of compression on said tensioning member (56).

12. The assembly of claim 11 wherein rotation of said spring housing (44) and said tamper member (40) with respect to each other permits said release of compression on said tensioning member (56).

13. The assembly of claim 8 wherein at least a portion of said tamper member (40) and said tensioning member (56) is contained in said spring housing (44).

14. The assembly of claim 13 wherein said tensioning member (56) is compressed in at least one of said spring housing (44) and said tamper member (40) in a first length of said spring housing (44) and said tamper member (40), and substantially uncompressed in a second length which is greater than said first length.

15. The assembly of claim 13 wherein said spring housing (44) and said tamper member (40) are rotatable with respect to each other to permit movement of said spring housing (44) and said tamper member (40) from a first length to a second length different from said first length.

16. The assembly of claim 14 wherein said tamper member (40) facilitates the distal movement of said second member (30) in the puncture when said spring housing (44) and said tamper member (40) are in said second length.

17. The assembly of claim 5 wherein said tamper member (40) applies distally directed pressure to said second member (30) in the puncture when said tensioning member (56) is in an uncompressed state.

## Patentansprüche

1. Anordnung zum Abdichten eines Schnitts oder einer Punktion im Körper eines Patienten, wobei die Punktion von der Haut des Patienten in ein Blutgefäß (26), einen Kanal oder einen Hohlraum des Patienten verläuft, wobei die Anordnung umfaßt:
ein erstes Bauteil (32), das aus bioabsorbierbarem Material gebildet und so dimensioniert ist, daß es sich in der Blutbahn (26), dem Kanal oder dem Hohlraum des Patienten positionieren und verankern läßt,
ein zweites Bauteil (30), das aus bioabsorbierbarem Material gebildet ist und zusammen mit dem ersten Bauteil (32) die Punktion gegenüber hindurchströmenden Flüssigkeiten abdichtet,
ein Fadenelement (34), das aus bioabsorbierbarem Material gebildet ist und einen Abschnitt aufweist, der proximal zu dem ersten Bauteil (32) und durch das zweite Bauteil (30) hindurch verläuft und dadurch das erste Bauteil (23) mit dem zweiten Bauteil (30) verbindet, und
eine Halteeinrichtung (20), die als Teil ein Spannelement (56) enthält, wobei das erste Bauteil (32), das zweite Bauteil (30) und das Fadenelement (34) in der Halteeinrichtung untergebracht sind,
**dadurch gekennzeichnet, daß** das Spannelement (56) mit dem Fadenelement (34) funktionsmäßig so verbunden ist, daß es auf das Fadenelement Spannung ausübt und dadurch bewirkt, daß das erste Bauteil (32) beim Abziehen des ersten Bauteils (32) und des zweiten Bauteils (30) von der Halteeinrichtung (20) in das abzudichtende Blutgefäß (26), den Kanal bzw. den Hohlraum eintritt und mit dessen Wand in Berührung kommt.

2. Anordnung nach Anspruch 1, mit ferner einem Stopfelement (40), wobei das Spannelement (56) Druckkräfte auf das Stopfelement (40) ausübt, die bewirken, daß sich das zweite Bauteil (30) in der Punktion distal in Richtung des ersten Bauteils (32) bewegt.

3. Anordnung nach Anspruch 1, wobei das Fadenelement (34) durch das zweite Bauteil (30) hindurchgeführt und mindestens mit dem ersten Bauteil (32), dem zweiten Bauteil (30) oder dem Spannelement (56) in Reibeingriff steht.

4. Anordnung nach Anspruch 1, wobei die Halteeinrichtung (20) ein Stopfelement (40) aufweist, durch das hindurch das Fadenelement (34) gleitend bewegbar ist.

5. Anordnung nach Anspruch 1 mit ferner einem länglichen Stopfelement (40), durch das das Fadenelement (34) hindurchgeführt ist.

6. Anordnung nach Anspruch 1 mit ferner einem das Spannelement (56) haltenden Federgehäuse (44).

7. Anordnung nach Anspruch 6, wobei das Fadenelement (34) durch mindestens einen Teil des Federgehäuses (44) hindurchgeführt ist.

8. Anordnung nach Anspruch 1, mit einem länglichen Federgehäuse (44) und einem länglichen Stopfelement (40), wobei das Spannelement (56) in dem Federgehäuse (44) gehalten und so orientiert ist, daß es eine distale Bewegung des Stopfelements (40) relativ zu dem Federgehäuse (44) hervorruft.

9. Anordnung nach Anspruch 8, wobei eine Drehung des Federgehäuses (44) relativ zu dem Stopfelement (40) eine Kraft hervorruft, die das Stopfelement (40) in der Punktion distal in Berührung mit dem zweiten Bauteil (30) bewegt.

10. Anordnung nach Anspruch 1, wobei die Halteeinrichtung (20) ein längliches Federgehäuse (44) und ein längliches Stopfelement (40) aufweist, wobei das Federgehäuse (44) und das Stopfelement (40) relativ zueinander derart orientiert sind, daß eine erste Länge vorhanden ist, in der das Spannelement (56) in dem Federgehäuse (44) gehalten ist und die Bewegung des Federgehäuses (44) und des Stopfelement (40) auf eine zweite Länge möglich ist, die größer ist als die erste Länge.

11. Anordnung nach Anspruch 10, wobei die Bewegung des Federgehäuses (44) und des Stopfelements (40) von der ersten auf die zweite Länge durch Freigeben von Druck auf das Spannelement (56) bewirkt wird.

12. Anordnung nach Anspruch 11, wobei eine relative Drehung zwischen dem Federgehäuse (44) und dem Stopfelement (40) die Freigabe von Druck auf das Spannelement (56) gestatten.

13. Anordnung nach Anspruch 8, wobei mindestens ein Teil des Stopfelements (40) und des Spannelements (56) in dem Federgehäuse (44) enthalten sind.

14. Anordnung nach Anspruch 13, wobei das Spannelement (56) in dem Federgehäuse (44) und/oder dem Stopfelement (40) in einer ersten Länge des Federgehäuses (44) und des Stopfelements (40) komprimiert und in einer zweiten Länge, die größer ist als die erste Länge, im wesentlichen nicht komprimiert ist.

15. Anordnung nach Anspruch 13, wobei das Federgehäuse (44) und das Stopfelement (40) relativ zueinander drehbar sind, um eine Bewegung des Federgehäuses (44) und des Stopfelements (40) von einer ersten Länge auf eine von dieser verschiedene zweite Länge gestattet.

16. Anordnung nach Anspruch 14, wobei das Stopfelement (40) die distale Bewegung des zweiten Bauteils (30) in der Punktion ermöglicht, wenn das Federgehäuse (44) und das Stopfelement (40) in der zweiten Länge vorliegen.

17. Anordnung nach Anspruch 5, wobei das Stopfelement (40) distal gerichteten Druck auf das zweite Bauteil (30) in der Punktion ausübt, wenn sich das Spannelement (56) in einem nicht komprimierten Zustand befindet.

## Revendications

1. Ensemble pour obturer une incision ou une piqûre dans le corps d'un patient, la piqûre s'étendant à partir de la peau du patient jusqu'à l'intérieur d'un vaisseau sanguin (26), d'un conduit ou d'une cavité du patient, l'ensemble comprenant :
un premier élément (32) constitué par un matériau bio-absorbable et dimensionné de façon à être positionné et ancré dans le vaisseau sanguin (26), le conduit ou la cavité du patient,
un deuxième élément (30) constitué par un matériau bio-absorbable pour obturer la piqûre vis-à-vis de l'écoulement de fluides à travers celle-ci en combinaison avec ledit premier élément (32),
un élément de filament (34) constitué par un matériau bio-absorbable et comprenant une partie s'étendant de façon proximale par rapport audit premier élément (32) et à travers ledit deuxième élément (30), de façon à interconnecter ainsi ledit premier élément (23) et ledit deuxième élément (30), et
un dispositif porteur (20) comprenant un élément de tension (56) faisant partie de celui-ci, ledit dispositif porteur recevant ledit premier élément (32), ledit deuxième élément (30) et ledit élément de filament (34) à l'intérieur de celui-ci,
**caractérisé en ce que** ledit élément de tension (56) est relié de façon opérationnelle audit élément de filament (34) de façon à appliquer une tension à celui-ci et à faire entrer ledit premier élément (32) dans la paroi du vaisseau sanguin (26), du conduit ou de la cavité devant être obturé et à le faire venir en contact avec celle-ci lors du déploiement dudit premier élément (32) et dudit deuxième élément (30) à partir dudit dispositif porteur (20).

2. Ensemble selon la revendication 1, comprenant de plus un élément de bourrage(40), ledit élément de tension (56) appliquant des forces de compression audit élément de bourrage (40) de façon à provoquer le déplacement de façon distale dudit deuxième élément (30) dans la piqûre vers ledit premier élément (32).

3. Ensemble selon la revendication 1, dans lequel ledit élément de filament (34) est enfilé à travers ledit deuxième élément (30) et vient en prise par frottement avec au moins l'un parmi ledit premier élément (32), ledit deuxième élément (30) et ledit élément de tension (56).

4. Ensemble selon la revendication 1, dans lequel ledit dispositif porteur (20) comprend de plus un élément de bourrage (40) et ledit élément de filament (34) peut coulisser à travers ledit élément de bourrage (40).

5. Ensemble selon la revendication 1, comprenant de plus un élément de bourrage allongé (40) à travers lequel est enfilé ledit élément de filament (34).

6. Ensemble selon la revendication 1, comprenant de plus un boîtier de ressort (44) maintenant ledit élément de tension (56).

7. Ensemble selon la revendication 6, dans lequel ledit élément de filament (34) est enfilé à travers au moins une partie dudit boîtier de ressort (44).

8. Ensemble selon la revendication 1, comprenant de plus un boîtier de ressort allongé (44) et un élément de bourrage allongé (40), ledit élément de tension (56) étant maintenu dans ledit boîtier de ressort (44) et orienté de façon à provoquer un déplacement distal dudit élément de bourrage (40) par rapport audit boîtier de ressort (44).

9. Ensemble selon la revendication 8, dans lequel la rotation dudit boîtier de ressort (44) par rapport audit élément de bourrage (40) provoque l'application d'une force déplaçant ledit élément de bourrage (40) de façon distale dans la piqûre en contact avec ledit deuxième élément (30).

10. Ensemble selon la revendication 1, dans lequel ledit dispositif porteur (20) comprend de plus un boîtier de ressort allongé (44) et un élément de bourrage allongé (40), ledit boîtier de ressort (44) et ledit élément de bourrage (40) étant orientés l'un par rapport à l'autre de façon à avoir une première longueur, ledit élément de tension (56) étant maintenu dans ledit boîtier de ressort (44), et permettant le déplacement dudit boîtier de ressort (44) et dudit élément de bourrage (40) sur une deuxième longueur qui est supérieure à ladite première longueur.

11. Ensemble selon la revendication 10, dans lequel le déplacement dudit boîtier de ressort (44) et dudit élément de bourrage (40) de ladite première longueur à ladite deuxième longueur est provoqué par un relâchement de la compression sur ledit élément de tension (56).

12. Ensemble selon la revendication 11, dans lequel la rotation dudit boîtier de ressort (44) et dudit élément de bourrage (40) l'un par rapport à l'autre permet ledit relâchement de la compression sur ledit élément de tension (56).

13. Ensemble selon la revendication 8, dans lequel au moins une partie dudit élément de bourrage (40) et dudit élément de tension (56) est contenue dans ledit boîtier de ressort (44).

14. Ensemble selon la revendication 13, dans lequel ledit élément de tension (56) est comprimé dans au moins l'un parmi ledit boîtier de ressort (44) et ledit élément de bourrage (40) dans une première longueur dudit boîtier de ressort (44) et dudit élément de bourrage (40), et sensiblement non comprimé dans une deuxième longueur qui est supérieure à ladite première longueur.

15. Ensemble selon la revendication 13, dans lequel ledit boîtier de ressort (44) et ledit élément de bourrage (40) peuvent tourner l'un par rapport à l'autre de façon à permettre le déplacement dudit boîtier de ressort (44) et dudit élément de bourrage 40) d'une première longueur à une deuxième longueur différente de ladite première longueur.

16. Ensemble selon la revendication 14, dans lequel ledit élément de bourrage (40) facilite le déplacement distal dudit deuxième élément (30) dans la piqûre lorsque ledit boîtier de ressort (44) et ledit élément de bourrage (40) sont dans ladite deuxième longueur.

17. Ensemble selon la revendication 5, dans lequel ledit élément de bourrage (40) applique une pression dirigée de façon distale audit deuxième élément (30) dans la piqûre lorsque ledit élément de tension (56) est dans un état non-comprimé.
